# EUROPEAN PATENT SPECIFICATION

(11) **EP 3 661 454 B1**
(45) Date of publication and mention of the grant of the patent: **14.09.2022**
(21) Application number: 18841759.6
(22) Date of filing: 25.07.2018
(51) Int. Cl.: A61F 2/07, A61F 2/82, A61L 31/16, C12N 15/11, A61L 27/54, A61L 29/16

(54) **DEVICE AND METHOD FOR PROMOTING RAPID STRUT COVERAGE AND VASCULAR ENDOTHELIAL COVERAGE**
VORRICHTUNG UND VERFAHREN ZUR FÖRDERUNG EINER SCHNELLEN STREBENENDOTHELISIERUNG UND GEFÄSSENDOTHELISIERUNG
DISPOSITIF ET PROCÉDÉ DESTINÉS À FAVORISER UNE COUVERTURE DE SUPPORT ET UNE COUVERTURE ENDOTHÉLIALE VASCULAIRE RAPIDES

(30) Priority: 31.07.2017 US 201762539356 P
(43) Date of publication of application: 10.06.2020
(73) Proprietor: Do Canto Zago, Alexandre, 90450-050 Porto Alegre - RS (BR)
(72) Inventor: DA SILVA ROSSATO, Juliane, 90450-050 Porto Alegre - RS (BR); ZAGO, Alcides Jose, 90450-050 Porto Alegre - RS (BR); PINHEIRO DO NASCIMENTO, Ludmila, 90450-050 Porto Alegre - RS (BR); DO CANTO ZAGO, Alexandre, 90450-050 Porto Alegre - RS (BR)
(74) Representative: Capitán García, Maria Nuria
(86) International application number: PCT/IB2018/055566
(87) International publication number: WO 2019/025908

(56) References cited:
- WO-A1-2013/152230
- WO-A1-2015/073274
- US-A1- 2006 115 454
- US-A1- 2009 117 169
- US-A1- 2016 058 923
- GARERI C. ET AL: "miR-125a-5p Modulates Phenotypic Switch of Vascular Smooth Muscle Cells by Targeting ETS-1", JOURNAL OF MOLECULAR BIOLOGY, vol. 429, no. 12, 1 June 2017 (2017-06-01) , pages 1817-1828, XP055787544, United Kingdom ISSN: 0022-2836, DOI: 10.1016/j.jmb.2017.05.008
- ARAKAWA EMI ET AL: "Benidipine, a Calcium Channel Blocker, Regulates Proliferation and Phenotype of Vascular Smooth Muscle Cells", JOURNAL OF PHARMACOLOGICAL SCIENCES, vol. 100, no. 2, 1 January 2006 (2006-01-01), pages 149-156, XP055787546, JP ISSN: 1347-8613, DOI: 10.1254/jphs.FPJ05024X
- BÄR HARALD ET AL: "Smoothelin is an indicator of reversible phenotype modulation of smooth muscle cells in balloon-injured rat carotid arteries", BASIC RESEARCH IN CARDIOLOGY, STEINKOPFF, DARMSTADT, DE, vol. 97, no. 1, 1 January 2002 (2002-01-01), pages 9-16, XP002497036, ISSN: 0300-8428, DOI: 10.1007/S395-002-8382-Z
- SMOLOCK et al.: "siRNA-mediated knockdown of h-caldesmon in vascular smooth muscle", Am J Physiol Heart Circ Physiol, vol. 297, no. 5, 18 September 2009 (2009-09-18), pages 1930-1939, XP055571999,
- YANG et al.: "Lentivirus-mediated RNAi targeting CREB binding protein attenuates neointimal formation and promotes re-endothelialization in balloon injured rat carotid artery", Cell Physiol Biochem, vol. 26, no. 3, 24 August 2010 (2010-08-24), pages 441-448, XP055572005,

## Description

### TECHNICAL FIELD

The invention generally relates to a field of a medical device and more particularly to a device and a method for promoting rapid strut coverage and vascular endothelial coverage.

### BACKGROUND OF THE INVENTION

Drug-eluting stents (DES) reduced significantly the rates of restenosis as compared to bare metal stents (BMS), but delayed significantly the stent re-endothelialization speed and increased the rates of stent thrombosis, thus requiring prolonged use of dual antiplatelet therapy (DAPT) to minimize such high thrombosis rates.

Therefore, DAPT duration increased from 30 days in case of BMS to 12 months when DES were used. Said DAPT long-term duration has clinical and financial impacts.

Among such clinical impacts are increased risk of bleeding, especially in patients predisposed to it, such as recent history of gastrointestinal bleeding, hemorrhagic stroke, arteriovenous malformations, tumors, etc., as well as patients in need of concomitant use of oral anticoagulants such as atrial fibrillation, atrial flutter, presence of prosthetic heart valve, thrombophilias, and/or recent history of deep vein thrombosis and/or pulmonary embolism among others.

Another important clinical aspect is a need for surgeries or dental procedures. Patients under DAPT cannot undergo surgical or dental procedures; therefore, a surgical or dental procedure should be postponed whenever possible up to the end of 12-month DAPT, which, more often than not, is not possible and requires the patient to discontinue DAPT for at least 5 days before surgery and, usually, restart it 2 days after medium or major surgery, thus increasing the rates of stent thrombosis over this period and, as a result, those of acute myocardial infarction due to stent occlusion.

Finally, hematomas are a frequent complaint mainly among elderly patients undergoing prolonged DAPT. In some cases, capillary fragility is such that a patient cannot tolerate it and DAPT has to be discontinued before the recommended period of time has elapsed.

As for the financial aspect, thienopyridines and P2Y12 receptor inhibitors are high-cost drugs; therefore, the financial impact on individuals and, especially, the public health care systems, become significant when DAPT is prolonged from 30 days in BMS to 12 months in DES.

Currently available DES deliver locally antiproliferative drugs as sirolimus or its analogs, that is, everolimus, biolimus, zotarolimus, and others. Paclitaxel is another class of antiproliferative drugs which was used in first generation DES, but then discontinued; however, this antiproliferative drug is at present used in practically all currently available DEB (drug-eluting balloon). US20160058923A discloses compositions, devices, grafts and methods for reducing or preventing anti-neointima following cardiovascular injuries and interventions, typically including an effective amount of a CTP synthase 1 inhibitor. C. Gareri et al. disclose that vascular SMCs phenotypic switch is one of the main events responsible for in-stent restenosis after percutaneous coronary intervention ("miR-125a-5p Modulates Phenotypic Switch of Vascular Smooth Muscle Cells by Targeting ETS-1", JOURNAL OF MOLECULAR BIOLOGY, vol. 429 (12), 01-06-2017, 1817-1828, XP055787544). Emi Arakawa et al. disclose that benipine reduces VSMCs hyperproliferation after balloon injury ("Benidipine, a Calcium Channel Blocker, Regulates Proliferation and Phenotype of Vascular Smooth Muscle Cells", JOURNAL OF PHARMACOLOGICAL SCIENCES, vol. 100 (2), 2006, 149-156, XP055787546). Harald Bär et al. disclose that after balloon angioplasty smoothelin is down-regulated during proliferation / neointima formation of dedifferentiated vascular SMCs ("Smoothelin is an indicator of reversible phenotype modulation of smooth muscle cells in balloon-injured rat carotid arteries", BASIC RESEARCH IN CARDIOLOGY, vol. 97 (1), 2002, 9-16, XP002497036).

Both sirolimus and its analogs and paclitaxel are antiproliferative agents with non-selective activity on a certain type of cells, that is, they inhibit proliferation of any kind of cells such as vascular smooth muscle cells (VSMC) and endothelial cells (EC) in a generic and extensive manner. Sirolimus and its analogs act by inhibiting cell proliferation in the G1-S cell cycle phase by binding to the intracellular receptor FK binding protein 12 (FKBP12) and consequently inhibiting the mammalian target of rapamycin (mTOR) pathway (Abe M, Kimura T, Morimoto T, Taniguchi T, Yamanaka F, Nakao K, et al. Sirolimus-eluting stent versus balloon angioplasty for sirolimus-eluting stent restenosis: Insights from the j-Cypher Registry. Circulation. 2010; 122:42-51). The mTOR pathway blocks cellular interphase, preventing the progression of the cell cycle from G1 to S, so without DNA synthesis the cell does not become able to perform mitosis (Yano H, Horinaka S, Yagi H, Ishimitsu T. Comparison of inflammatory response after implantation of sirolimus- and paclitaxel-eluting stents in patients on hemodialysis. Heart Vessels. 2012; Curcio A, Torella D, Indolfi C. Mechanisms of smooth muscle cell proliferation and endothelial regeneration after vascular injury and stenting: approach to therapy. Circ J. 2011; 75:1287-96). Paclitaxel also promotes cell cycle blockade but through a different mechanism than sirolimus. Paclitaxel acts in the G2-M phase of the cell cycle, preventing the cell passing from the G2 phase of the interphase to the onset of mitosis (Cheng H, An SJ, Zhang XC, Dong S, Zhang YF, Chen ZH, et al. In vitro sequence-dependent synergism between paclitaxel and gefitinib in human lung cancer cell lines. Cancer Chemother Pharmacol. 2011; 67:637-46). That being so, paclitaxel acts on the formation of microtubules, which are fundamental elements for the formation of the mitotic spindle and orientation of the chromosomes during the process of cell division (Pires NM, Eefting D, de Vries MR, Quax PH, Jukema JW. Sirolimus and paclitaxel provoke different vascular pathological responses after local delivery in a murine model for restenosis on underlying atherosclerotic arteries. Heart. 2007; 93:922-7).

The search for the ideal medical device has been ongoing ever since the coronary interventions with balloon catheters began. Several medical devices such as atheroablation devices (directional atherectomy and rotational atherectomy - rotablator) as well as metallic stents were evaluated in the 90's, the metallic stents having shown the best clinical results when compared with balloon, directional atherectomy, and rotablator. In spite of progress made in increasing procedure success rates and reducing restenosis rates thanks to the advent of BMS, in-stent restenosis (ISR) remained high varying from 20 to 50%, depending on a patient's clinical and angiographic characteristics.

A new significant breakthrough was achieved in the early 2000s with the advent of DES that reduced significantly ISR; nevertheless, said DES delayed significantly stent re-endothelialization and, as a result, increased stent thrombosis rates and consequently duration of DAPT. Therefore, the search for a medical device that ensures simultaneously both permanent patency of the blood vessel and early re-endothelialization of the treated vessel segment, that is, which is capable of inhibiting VSMC proliferation and not interfering with or even stimulating EC production and local deposition continues to be necessary.

Since antiproliferative drugs such as sirolimus and/or analogs and paclitaxel delay the reestablishment of the endothelium in the inner stent surface, this lack of a protective mechanism in the vascular wall allows the development of neointimal hyperplasia (Tarantini G, Facchin M, Capodanno D, Musumeci G, Saia F, Menozzi A, et al. Paclitaxel versus sirolimus eluting stents in diabetic patients: does stent type and/or stent diameter matter?: long-term clinical outcome of 2,429-patient multicenter registry. Catheter Cardiovasc Interv. 2013; 81:80-9). Studies show that early re-endothelialization prevents restenosis; therefore, a DES that elutes a drug capable of inhibiting VSMC and promotes rapid re-endothelialization would probably offer additional benefits with potentially higher superiority in the inhibition of VSMC over antiproliferative drugs used in current DES. The endothelium plays an important role in the regulation of vascular tone and in the maintenance of cardiovascular homeostasis, which is determinative of the control of thrombolysis, vascular remodeling and inflammatory response by the activation of the immune system (Luz PL. Endotélio e doenças cardiovasculares. São Paulo: Ateneu; 2005.; Glasser SP, Selwyn AP, Ganz P. Atherosclerosis: risk factors and the vascular endothelium. Am Heart J. 1996; 131:379-84).

The currently available DES show average ISR of only 8% by means of controlled neointima formation through the inhibition of VSMC hyperproliferation. Such low ISR rate is mainly attributed to mechanical issues such as stent malapposition, undersized stent, or underexpanded stent. Therefore, the most important issue regarding the improvement of current DES relies on stent re-endothelialization since these antiproliferative drugs inhibit both VSMC and EC. Thus, such stent re-endothelialization delay causes significant clinical and financial negative impacts as already mentioned above.

Therefore, a drug capable or inhibiting VSMC without interfering with the proliferation of EC to create a medical device that promotes rapid struts and/or vascular endothelial coverage is currently required. The two gene silencers myosin heavy chain 11 (MYH11)-siRNA and calponin 1 (CNN1)-siRNA are identified as the most effective drugs for a medical device used for desobstructing blood vessels. The main advantages of a MYH11 -siRNA-eluting or a CNN1 - siRNA-eluting medical device is that such gene silencers-eluting medical devices simultaneously prevent vascular reobstruction due to the efficient inhibition of VSMC hyperproliferation and promote early re-endothelialization since the gene silencers MYH11-siRNA and CNN1-siRNA do not inhibit the proliferation of EC.

In additional experiments, it was found that the gene silencers leiomodin 1 (LMOD)-siRNA, smoothelin (SMTN)-siRNA, tropomyosin (TPM)-siRNA, caldesmon 1 (CALD)-siRNA, actinin (ACTN)-siRNA, actin alpha (ACTA)-siRNA and actin beta (ACTB)-siRNA are also effective in inhibiting VSMC hyperproliferation as well as in promoting early re-endothelialization since such gene silencers also do not inhibit significantly the proliferation of EC. Therefore, in addition to the MYH11-siRNA-eluting and CNN1-siRNA-eluting medical devices, LMOD-siRNA-eluting, SMTN-siRNA-eluting, TPM-siRNA-eluting, CALD-siRNA-eluting, ACTA-siRNA-eluting, ACTN-siRNA-eluting and ACTB-siRNA-eluting medical devices are also included.

### SUMMARY OF THE INVENTION

The invention is defined by the claims. Any subject matter falling outside the scope of the claims is provided for information purposes only.

The invention overcomes the above problem by introducing a device according to claim 1 and a device for use in a method for promoting rapid strut coverage and vascular endothelial coverage according to claim 10. The invention inhibits VSMC hyperproliferation and promotes early re-endothelialization of blood vessels.

In view of the foregoing, an embodiment herein provides a device and a device for use in a method for inhibiting VSMC hyperproliferation and promoting early re-endothelialization of blood vessels.

An embodiment herein provides a device for promoting rapid strut coverage and vascular endothelial coverage. The device comprises an eluting medical device and at least one gene silencer. The eluting device is coated or filled in with at least one gene silencer. The gene silencer is selected from myosin heavy chain 11 (MYH11)-siRNA, calponin 1 (CNN1)-siRNA, leiomodin 1 (LMOD)-siRNA, smoothelin (SMTN)-siRNA, tropomyosin (TPM)-siRNA, caldesmon 1 (CALD)-siRNA, actinin (ACTN)-siRNA, actin alpha (ACTA)-siRNA, and actin beta (ACTB)-siRNA. The gene silencer is delivered locally in blood vessel to enter the vessel wall and VSMC for the selective inhibition of the VSMC hyperproliferation without inhibiting EC proliferation.

In the embodiment related to the device mentioned in paragraph [018], the device comprises a gene silencer vehicle which facilitates elution of the gene silencer. The gene silencer vehicle allows the gene silencer to enter the blood vessel wall and vascular smooth muscle cell. In the same embodiment, the gene silencer vehicle is selected from a group of lentiviruses and plasmids, nanoparticles, fluoropolymers, bioabsorbable polymers, non-absorbable polymers, or can even be polymer free (without any kind of polymer).

In the embodiment related to the device mentioned in paragraph [018], the eluting medical device is selected from any implantable device and non-implantable local drug delivery device.

In the embodiment related to the device mentioned in paragraph [018], the implantable device is selected from a metallic stent and a bioresorbable vascular scaffold (BVS).

In the embodiment related to the device mentioned in paragraph [018], the non-implantable local drug delivery device is selected from DEB, local drug delivery catheter and any other medical device capable of locally delivering MYH11-siRNA, CNN1-siRNA, LMOD-siRNA, SMTN-siRNA, TPM-siRNA, CALD-siRNA, ACTN-siRNA, ACTA-siRNA, and ACTB-siRNA.

In the embodiment related to the device mentioned in paragraph [018], the gene silencer is delivered alone or in combination with another siRNA or with cell antiproliferative drugs.

In the embodiment related to the device mentioned in paragraph [018], the gene silencer concentration ranges from 1 µg/ml to 1000 µg/ml, which is a wide concentration range, since the gene silencer can be spread over the medical device surface from a single very thin layer up to several thick layers to reach the desired amount of gene silencer to be locally delivered.

An embodiment herein describes an eluting medical device for use in a method for promoting rapid strut coverage and vascular endothelial coverage. The method comprises:
a) an eluting medical device and at least one gene silencer; and
b) an eluting medical device coated or filled in with at least one gene silencer;
c) the gene silencer is selected from: myosin heavy chain 11 (MYH11)-siRNA, calponin 1 (CNN1)-siRNA, leiomodin 1 (LMOD)-siRNA, smoothelin (SMTN)-siRNA, tropomyosin (TPM)-siRNA, caldesmon 1 (CALD)-siRNA, actinin (ACTN)-siRNA, actin alpha (ACTA)-siRNA, and actin beta (ACTB)-siRNA;
d) the gene silencer promotes early re-endothelialization of the blood vessel segment inhibiting only VSMC hyperproliferation, thus the gene silencer does not significantly interfere with EC production or may even stimulate it;
e) the gene silencer is delivered to a target segment of the blood vessel;
f) the gene silencer can be loaded into a gene silencer vehicle;
g) the gene silencer vehicle facilitates elution of the gene silencer;
h) the gene silencer vehicle allows the gene silencer to enter the targeted blood vessel wall and vascular smooth muscle cells; and
i) the gene silencer can be loaded directly onto the device surface (without vehicle) or even into the device.

In one of the embodiments, the method facilitates both permanent patency of the blood vessel and early re-endothelialization of a treated vessel segment.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation.

### BRIEF DESCRIPTION OF THE DRAWINGS

FIG. 1 describes VSMC proliferation in bottles containing human VSMC according to the following treatment groups, the gene silencers vehicle being lentivirus and plasmid;
FIG. 2 describes an EC proliferation in bottles containing human EC according to the following treatment groups, the gene silencers vehicle being lentivirus and plasmid;
FIG. 3 describes VSMC proliferation in bottles containing human VSMC according to the following treatment groups, the gene silencers vehicle being lentivirus and plasmid;
FIG. 4 describes EC proliferation in bottles containing human EC according to the following treatment groups, the gene silencers vehicle being lentivirus and plasmid;
FIG. 5 describes VSMC proliferation in bottles containing human VSMC according to the following treatment groups, the gene silencers vehicle being lentivirus and plasmid;
FIG. 6 describes VSMC proliferation in bottles containing human VSMC according to the following treatment groups, the vehicle of gene silencers and cell antiproliferative drugs being nanoparticles; and
FIG. 7 describes EC proliferation in bottles containing human EC according to the following treatment groups, the vehicle of gene silencers and cell antiproliferative drugs being nanoparticles.
Fig. 8 describes VSMC proliferation in bottles containing human VSMC according to the following treatment groups, the vehicle of gene silencers and cell antiproliferative drugs being nanoparticles.
Fig. 9 describes EC proliferation in bottles containing human EC according to the following treatment groups, the vehicle of gene silencers and cell antiproliferative drugs being nanoparticles.
Fig. 10 describes VSMC proliferation in bottles containing human VSMC according to the following treatment groups in different escalating doses, the vehicle of gene silencers and cell antiproliferative drugs being nanoparticles.
Fig. 11 describes EC proliferation in bottles containing human EC according to the following treatment groups in different escalating doses, the vehicle of gene silencers and cell antiproliferative drugs being nanoparticles.

### DETAILED DESCRIPTION OF PREFERRED EMBODIMENTS

The embodiments herein and the various features and advantageous details thereof are explained more fully with reference to the non-limiting embodiments that are illustrated in the accompanying drawings and detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

The embodiments herein achieve this by providing a device and an eluting device for use in a method for inhibiting VSMC hyperproliferation and promoting early re-endothelialization of blood vessels. Attempts to develop a medical device which provides both permanent patency of the blood vessel and early re-endothelialization of the treated vessel segment, that is, a device capable of inhibiting VSMC hyperproliferation and not interfering with or even stimulating EC production and local deposition, have been made ever since the advent of Interventional Cardiology.

Current DES elute antiproliferative drugs that reduced significantly the restenosis rates as compared to BMS; nevertheless, the benefits of DES were achieved at the expense of significantly delayed stent re-endothelialization and, as a result, increased stent thrombosis rates and, consequently, prolonged duration of DAPT. Therefore, only part of the problem was properly solved with currently available DES.

Thus, a medical device that ensures simultaneously both permanent patency of the blood vessel, as current DES do, and early re-endothelialization of the vessel treated segment, which remains to be a problem for current DES, is required. That is, the search for a medical device capable of both inhibiting VSMC proliferation and migration towards the inner surface of the vessel, which causes vessel lumen obstruction, and not interfering with or even stimulating EC production and local deposition.

In one of the embodiments, a search for gene targets was conducted in order to identify an agent capable of significantly inhibiting VSMC hyperproliferation and, simultaneously, not interfering with EC proliferation. Therefore, a clinical study was performed on tissue fragments from restenotic plaques in Experiment 1 which identified two potential gene targets. Thus, the tumor necrosis factor receptor superfamily, member 11b (TNFRSF11B)-siRNA and neutrophil cytosolic factor 4 (NCF4)-siRNA gene silencers were assessed in Experiment 2, which was an in vitro experiment, that compared TNFRSF11B-siRNA and NCF4-siRNA gene silencers to antiproliferative drugs (sirolimus and paclitaxel) proved to have an inhibitory effect on VSMC hyperproliferation as well as to control gene silencers with no inhibitory effect on VSMC and EC previously described in the literature or even with no theoretical rationale to act in an inhibitory manner on VSMC and EC proliferation (smooth muscle protein 22a (SM22a)-siRNA and MYH11-siRNA).

The embodiments herein provide the results of Experiment 2 in which the two gene silencers - TNFRSF11B-siRNA and NCF4-siRNA - do not demonstrate the expected benefits while one of the gene silencers used as a control of the lentivirus vehicle - MYH11-siRNA - shows significant inhibition of VSMC proliferation. Then, Experiment 3 was performed in order to confirm the results from Experiment 2. Finally, several other gene silencers of VSMC structural and functional proteins were tested in Experiment 4 (MYH11-siRNA, CNN1-siRNA, myosin light chain 9 (MYL9)-siRNA, TRIO and F-actin binding protein (TRIOBP)-siRNA, myosin light chain kinase (MYLK)-siRNA, and myosin phosphatase Rho interacting protein (MPRIP)-siRNA), being CNN1-siRNA and MYH11 - siRNA the only gene silencers that show significant inhibition of VSMC proliferation.

As for EC, the TNFRSF11B-siRNA and NCF4-siRNA gene silencers do not cause significant inhibition of their proliferation, which is already expected, since the goal in relation to these therapeutic targets is not to interfere with or interfere as little as possible with EC proliferation. The other gene silencers assessed as controls, the MYH11-siRNA and CNN1-siRNA among them, did not show significant inhibition of EC proliferation either, which had already been expected since these gene silencers were chosen as controls because, according to the data available in the literature, they interfere neither with VSMC nor with EC proliferation. The significant EC inhibition in the sirolimus and paclitaxel groups is well known and described in the literature, as already discussed above when the problem of stent thrombosis was dealt with.

The use of lentiviruses and plasmids for transfection of gene silencers into cells is a common and demonstrably effective method. However, this method may be difficult to apply to the manufacturing of DES, BVS, DEB, and other devices which are often sterilized by processes that involve high temperatures, such as ethylene oxide (ETO). Therefore, in Experiment 5 nanoparticles were assessed as gene silencer carriers with satisfactory results.

Experiment 6 was the first in vivo study to evaluate and prove the efficacy of both MYH11 -siRNA-eluting stent and CNN1 -siRNA-eluting stent in promoting sustained inhibition of in-stent hyperplasia (like sirolimus-eluting stent), as well as in allowing rapid stent struts re-endothelialization (like a bare metal stent) through a mechanism of action that is not described in the medical literature.

Then, based on the very promising results from MYH11-siRNA and CNN1-siRNA, additional 32 gene silencers related to structural and functional proteins of human VSMC were evaluated in in vitro Experiment 7 which revealed the efficacy of LMOD-siRNA, SMTN-siRNA, TPM-siRNA, CALD-siRNA, ACTN-siRNA, ACTA-siRNA and ACTB-siRNA in significantly inhibiting human VSMC proliferation without interfering with the proliferation of human EC. However, Experiment 7 also showed that the 9 effective gene silencers identified have different potency or efficacy in inhibiting human VSMC when using the same doses.

Finally, in order to assess the relation between gene silencer dosage and potency/efficacy in inhibiting human VSMC, Experiment 8 was performed and showed that the gene silencers have a wide range of doses at which they are effective and its effectiveness is not dose-dependent. Moreover, high or very high doses of gene silencers do not interfere with the proliferation of human EC.

Therefore, in one of the embodiments, the gene silencer concentration ranges from 1 µg/ml to 1000 µg/ml, which is a wide concentration range, since the gene silencer can be spread over the medical device surface from a single very thin layer up to several thick layers to reach the desired amount of gene silencer to be locally delivered.

The 8 experiments described herein showed evidence that MYH11-siRNA, CNN1-siRNA, LMOD-siRNA, SMTN-siRNA, TPM-siRNA, CALD-siRNA, ACTN-siRNA, ACTA-siRNA and ACTB-siRNA are the only gene silencers capable of inhibiting VSMC proliferation in an unassailable manner, which is not a class effect of the gene silencers of VSMC structural and functional proteins because all the other gene silencers tested that belong to these classes of gene silencers are not able to show the same effect on inhibiting VSMC proliferation.

Consequently, all the 9 gene silencers MYH11-siRNA, CNN1-siRNA, LMOD-siRNA, SMTN-siRNA, TPM-siRNA, CALD-siRNA, ACTN-siRNA, ACTA-siRNA and ACTB-siRNA have a very particular effect on human VSMC inhibition, which is not extendable to all of the other gene silencers of their class. This specific effect of MYH11-siRNA, CNN1-siRNA, LMOD-siRNA, SMTN-siRNA, TPM-siRNA, CALD-siRNA, ACTN-siRNA, ACTA-siRNA and ACTB-siRNA are their activity against one or more key proteins in the VSMC proliferation process, that is, the inhibition of one or more proteins fundamental for the formation of young VSMC, since all the 9 proteins MYH11, CNN1, LMOD, SMTN, TPM, CALD, ACTN, ACTA and ACTB belong to mature VSMC. Thus, to understand ISR, a possible plasticity developed by quiescent cells may result from a cell phenotypic change allowing the genes previously activated only in the embryonic period to start expressing again, generating a high proliferative potential. Another possible mechanism is the activity of MYH11-siRNA, CNN1-siRNA, LMOD-siRNA, SMTN-siRNA, TPM-siRNA, CALD-siRNA, ACTN-siRNA, ACTA-siRNA and ACTB-siRNA against one or more proteins or their mechanisms of action.

The results regarding the inhibition of VSMC proliferation by MYH11-siRNA, CNN1-siRNA, LMOD-siRNA, SMTN-siRNA, TPM-siRNA, CALD-siRNA, ACTN-siRNA, ACTA-siRNA and ACTB-siRNA not only raise several questions but also bring some certainties such as the fact that the ISR mechanism is much more complex than previously thought and that one or more metabolic pathways silenced by MYH11-siRNA, CNN1-siRNA, LMOD-siRNA, SMTN-siRNA, TPM-siRNA, CALD-siRNA, ACTN-siRNA, ACTA-siRNA and ACTB-siRNA are of extreme importance for VSMC hyperproliferation.

In another embodiment, the medical device consists of:
a) MYH11-siRNA- eluting medical device;
b) CNN1-siRNA- eluting medical device,
c) LMOD-siRNA- eluting medical device,
d) SMTN-siRNA- eluting medical device,
e) TPM-siRNA- eluting medical device,
f) CALD-siRNA- eluting medical device,
g) ACTN-siRNA- eluting medical device,
h) ACTA-siRNA- eluting medical device; and
i) ACTB-siRNA-eluting medical device
wherein all of these gene silencers do not significantly interfere with the proliferation of human EC. Therefore, differently from the DES which elute cell antiproliferative drugs such as sirolimus or analogs and paclitaxel that inhibit human vascular EC proliferation and consequently delay early stent re-endothelialization, all of the following MYH11-siRNA-, CNN1-siRNA-, LMOD-siRNA-, SMTN-siRNA-, TPM-siRNA-, CALD-siRNA-, ACTN-siRNA-, ACTA-siRNA- and ACTB-siRNA-eluting medical devices being capable of promoting early stent, BVS or vessel re-endothelialization.

Another important aspect related to early re-endothelialization is the studies that show potential benefits of early re-endothelialization with regard to ISR; thus, early re-endothelialization stimulated by MYH11 -siRNA-, CNN1-siRNA-, LMOD-siRNA-, SMTN-siRNA-, TPM-siRNA-, CALD-siRNA-, ACTN-siRNA-, ACTA-siRNA- and ACTB-siRNA-eluting medical device is capable of promoting additional benefits in the inhibition of VSMC hyperproliferation in comparison with the currently available cell antiproliferative drug-eluting medical devices. Since cell antiproliferative drugs such as sirolimus and its analogs and paclitaxel delay the reestablishment of the endothelium in the inner stent surface, this lack of a protective mechanism in the vascular wall enables the development of neointimal hyperplasia and, as a result, ISR.

The gene silencer vehicle used for local delivery of any of all the 9 gene silencers may be any vehicle that allows MYH11-siRNA, CNN1-siRNA, LMOD-siRNA, SMTN-siRNA, TPM-siRNA, CALD-siRNA, ACTN-siRNA, ACTA-siRNA and ACTB-siRNA to enter human VSMC; therefore, it may be anything from lentiviruses and plasmids, nanoparticles, fluoropolymers, bioabsorbable polymers, and non-absorbable polymers to polymer free MYH11-siRNA coated medical device, polymer free CNN1-siRNA coated medical device, polymer free LMOD-siRNA coated medical device, polymer free SMTN-siRNA coated medical device, polymer free TPM-siRNA coated medical device, polymer free CALD-siRNA coated medical device, polymer free ACTN-siRNA coated medical device, polymer free ACTA-siRNA coated medical device, and polymer free ACTB-siRNA coated medical device, or yet any other medical device vehicle since the most important thing is to ensure that the gene silencers MYH11-siRNA, CNN1-siRNA, LMOD-siRNA, SMTN-siRNA, TPM-siRNA, CALD-siRNA, ACTN-siRNA, ACTA-siRNA and/or ACTB-siRNA enter the vessel wall and VSMC.

The eluting medical device must be coated or filled in with MYH11-siRNA, CNN1-siRNA, LMOD-siRNA, SMTN-siRNA, TPM-siRNA, CALD-siRNA, ACTN-siRNA, ACTA-siRNA or ACTB-siRNA plus preferably but not necessarily a vehicle, the medical device being both any implantable device such as a metallic stent, BVS, and/or other implantable device or any non-implantable local drug delivery device such as a DEB, local drug delivery catheter and/or other medical device capable of locally deliver MYH11-siRNA, CNN1-siRNA, LMOD-siRNA, SMTN-siRNA, TPM-siRNA, CALD-siRNA, ACTN-siRNA, ACTA-si RNA and ACTB-siRNA.

The gene silencers MYH11 -siRNA, CNN1-siRNA, LMOD-siRNA, SMTN-siRNA, TPM-siRNA, CALD-siRNA, ACTN-siRNA, ACTA-siRNA and ACTB-siRNA can be locally delivered alone, together or in combination with another siRNA and/or cell antiproliferative drugs and/or other drugs, since the main mechanism of action, which is the selective and significant inhibition of the VSMC hyperproliferation without inhibiting significantly EC proliferation, is based on the gene silencers MYH11-siRNA, CNN1-siRNA, LMOD-siRNA, SMTN-siRNA, TPM-siRNA, CALD-siRNA, ACTN-siRNA, ACTA-siRNA and ACTB-siRNA.

The identification and evaluation of new drugs with selective and efficient VSMC inhibitory activity as well as with no interference with EC proliferation were done through a clinical study (Experiment 1), some in vitro studies (Experiments 2,3,4,5,7 and 8) and an in vivo study (Experiment 6) to support the invention.

### Experiment 1

Out of 27 patients who underwent directional atherectomy with the Flexicut (Guidant, USA) catheter to withdraw fragments of ISR plaques and de novo coronary artery plaques, 12 patients had ISR and 15 patients had symptomatic de novo coronary artery lesions. The atherectomy samples (plaques) were identified, placed in RNA Stat 60 and stored in liquid nitrogen until gene profiling analysis of more than 22,000 genes. RNA was isolated from atherectomy samples by homogenization with a PowerGen model 125 homogenizer (Thermo Fisher Scientific, USA), followed by extraction with 0.2 volume of chloroform, precipitation with 0.5 volume of isopropanol, wash in 75% ethanol, and suspension in RNase-free water. RNA concentration was measured by use of the NanoDrop spectrophotometer (Thermo Fisher Scientific, USA). RNA quality and purity were assessed by the RNA 6000 pico or nano assay using Agilent 2100 Bioanalyzer (Agilent Technologies, USA).

Samples were labelled for gene expression profiling using the Affymetrix Gene Chip microarray system (Affymetrix, USA). A standard T7 based amplification protocol (Affymetrix) was used to convert RNA to biotinylated cRNA. Fragmented, biotin-labeled cRNA was hybridized to Affymetrix Gene Chip Human Genome U133A arrays according to standard Affymetrix protocol. Operators, chip lots, and scanners (GeneArray 3000, Affymetrix) were controlled throughout. Quality controls of arrays that used GeneChip Operating Software included scaling factor and percentage of genes present.

Gene expression profiling of ISR plaques were compared with gene expression profiling of de novo coronary artery plaques; only the genes expressed more than 15X in the ISR plaques were considered significant.

Two genes were found to be expressed more than 15X, that is, the tumor necrosis factor receptor super family, member 11b (TNFRSF11B) which was expressed 18X more in the ISR plaques in comparison with de novo coronary artery plaques, and the neutrophil cytosolic factor 4 (NCF4) which was expressed 16X more in the ISR plaques.

The TNFRSF11B gene encodes a member of the tumor necrosis factor (TNF) cytokine family, which is a ligand for osteoprotegerin and functions as a key factor for osteoclast differentiation and activation. This protein was shown to be a dentritic cell survival factor and is involved in the regulation of T cell-dependent immune response. T cell activation was reported to induce expression of this gene and lead to an increase of osteoclasto genesis and bone loss. This protein was shown to activate anti-apoptotic kinase AKT/PKB through a signaling complex involving SRC kinase and tumor necrosis factor receptor-associated factor 6 (TRAF 6), which indicated that this protein may have a role in the regulation of cell apoptosis.

The NCF4 encodes a protein that is a cytosolic regulatory component of the superoxide-producing phagocyte NADPH-oxidase, a multicomponent enzyme system important for host defense. This protein is preferentially expressed in cells of myeloid lineage. It interacts primarily with neutrophil cytosolic factor 2 (NCF2/p67-phox) to form a complex with neutrophil cytosolic factor 1 (NCF1/p47-phox), which further interacts with the small G protein RAC1 and translocates to the membrane upon cell stimulation.

### Experiment 2

After identifying 2 genes with a high potential for modulating the inflammatory response that causes ISR in Experiment 1, the therapeutic response was assessed by neutralizing said 2 genes (TNFRSF11B & NCF4) in bottles containing human VSMC or human vascular EC by means of gene silencers which are a specific interfering RNA sequence that block the protein translation of a specific gene; in this case, that of the NFRSF11B and NCF4 genes.

Therefore, Experiment 2 was carried out with the following groups: lentivirus + gene silencer TNFRSF11B-siRNA, lentivirus + gene silencer NCF4-siRNA, full control (no vehicle and no drug), lentivirus + gene silencer SM22a-siRNA + glyceraldehyde 3-phosphate dehydrogenase (GAPDH) (vehicle control 1), lentivirus + gene silencer MYH11-siRNA + GAPDH (vehicle control 2), sirolimus (drug control 1), and paclitaxel (drug control 2).

TNFRSF11B-siRNA and NCF4-siRNA are gene silencers of the TNFRSF11B and NCF4 genes, respectively. The delivery system of gene silencers was developed by inserting the respective gene silencers in a plasmid which is carried by a lentivirus. Lentivirus particles are intracellular transduction-ready viruses containing plasmids with gene silencers. After transduction, cells express permanently gene silencers. The density of cell cultures 24 hours before transfection was 2x105 cells/10 cm2 in culture medium. As a result, 5µL aliquots from 10-1000 µM of siRNA in 100 µL/mL of transfection culture were taken.

Two control groups containing a lentivirus and a gene silencer of smooth muscle protein 22a (SM22a) or smooth muscle myosin heavy chain (MYH11) were used to assess the vehicle. Said two genes were chosen because they are characterized as markers of the presence of differentiated (mature) VSMC, that is, they are identified only in VSMC at the final stage of differentiation, said cells being considered as quiescent. Therefore, there is no association between these 2 genes and VSMC proliferation modulation mechanisms in the literature, nor are they young VSMC markers. Thus, said two gene silencers, SM22a-siRNA and MYH11-siRNA, were marked with glyceraldehyde 3-phosphate dehydrogenase (GAPDH), called bioconjugates, to ensure by means of fluorescence microscopy that the gene silencers effectively entered VSMC and EC. Also, these two vehicle controls were used to show that the vehicle does not interfere with VSMC and EC proliferation processes. The doses of SM22a-siRNA and MYH11-siRNA were also similar to those of the previous gene silencers, that is, 5µL aliquots from 10 - 1000 µM of siRNA in 100 µL/mL of transfection medium were taken.

The two drug control groups consisted of sirolimus and paclitaxel, respectively. Sirolimus and paclitaxel were prepared in stock solutions with ethanol (20mg/dL) and diluted in culture medium in working solutions ranging from 10 - 1000 micrograms/mL. Said two drugs belong to the only two classes of drugs of proven VSMC inhibition efficiency in clinical practice by means of DES that also inhibit significantly EC proliferation, having negative effects on stent re-endothelialization.

In the full control group, VSMC were cultured in smooth muscle cell growth medium (Gibco, medium 231) while EC were cultured in endothelial cell medium (Gibco, medium 200). Both were supplemented with 10% fetal bovine serum (FBS, Invitrogen, Calsbad, CA, USA), streptomysin (100 µg/mL) and penicillin (100U/mL). Therefore, neither vehicle nor drug was used.

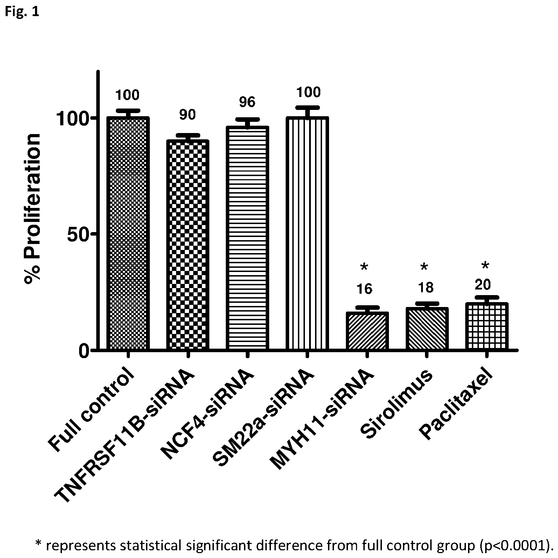

The results of the bottles containing human VSMC are shown in Figure 1.

The data on the VSMC culture showed no statistically significant difference in cell proliferation of the TNFRSF11B-siRNA, NCF4-siRNA, and SM22a-siRNA groups as compared to the full control group. There was also statistically significant inhibition in cell proliferation of the sirolimus, paclitaxel, and MYH11-siRNA groups in relation to the full control group, but no statistically significant difference between these 3 groups. The statistically significant difference between the sirolimus and paclitaxel groups and the full control group as well as the lack of significant difference between the SM22a-siRNA group and the full control group had already been expected. However, the MYH11-siRNA promoted a statistically significant inhibition of VSMC proliferation, that is, the silencing of a gene expressed only in mature/differentiated VSMC interfered with the proliferation of young VSMC, inhibiting significantly their proliferation with magnitude similar to that of sirolimus and paclitaxel.

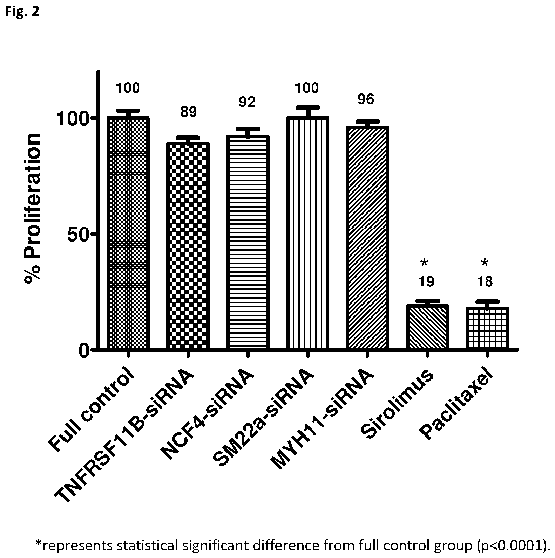

The results of the bottles containing human vascular EC are shown in Figure 2. As for the data on the EC culture, the results showed no statistically significant difference in cell proliferation of the TNFRSF11B-siRNA, NCF4siRNA, SM22a-siRNA, and MYH11-siRNA groups in relation to the full control group. There was also statistically significant inhibition in cell proliferation between the sirolimus and paclitaxel groups and the full control group. The lack of statistical difference in the TNFRSF11B-siRNA and NCF4-siRNA groups had already been expected since the goal of these therapeutic targets was not to interfere or interfere as little as possible with the EC proliferation. The lack of the significant difference between the SM22a-siRNA and MYH11siRNA groups had already been expected too because these two gene silencers were chosen as control since they supposedly do not interfere with both VSMC and EC cell proliferation according to the data available in the literature. The significant EC inhibition in the sirolimus and paclitaxel groups is already well known and described in the literature as discussed above when the problem of stent thrombosis was dealt with.

The MYH11-siRNA significantly inhibited VSMC proliferation in comparison with the full control. Although MYH11-siRNA inhibited human VSMC numerically more than sirolimus and paclitaxel, there was no significant difference between all of these 3 groups. The MYH11-siRNA inhibition of human vascular EC was comparable to the full control and extremely lower than sirolimus and paclitaxel.

The TNFRSF11B-siRNA and NCF4-siRNA failed to significantly inhibit VSMC proliferation in comparison with the full control.

### Experiment 3

In Experiment 2, the 2 gene silencers identified in Experiment 1 - TNFRSF11B-siRNA and NCF4-siRNA - did not show the expected benefits while one of the two gene silencers used as a control of the lentivirus vehicle - MYH11 -siRNA - showed what was expected from the TNFRSF11B-siRNA and NCF4-siRNA, that is, significant inhibition of VSMC proliferation and non-significant inhibition of EC proliferation.

Therefore, even though the probability of error when identifying bottles or replacing gene silencers while preparing the suspensions was low as a result of the team's careful work and experience, it was decided to partially repeat Experiment 2 to confirm the results obtained in relation to the MYH11-siRNA.

Thus, Experiment 3 consisted of the following groups: lentivirus + gene silencer MYH11-siRNA, lentivirus + gene silencer TNFRSF11BsiRNA, and lentivirus + gene silencer NCF4-siRNA.

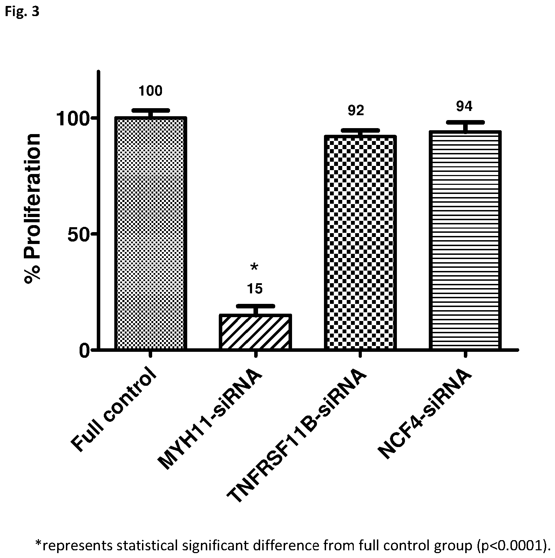

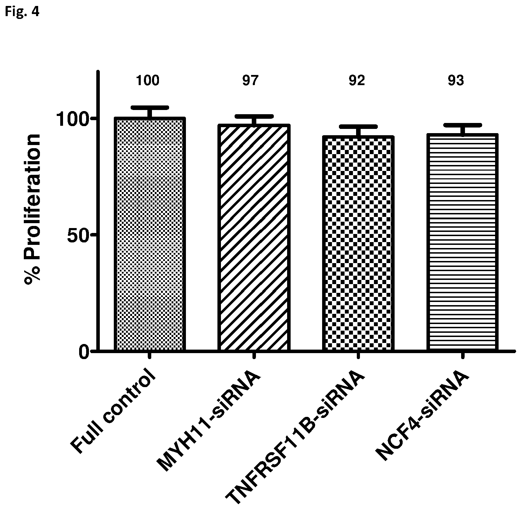

The results of the bottles containing human VSMC and human vascular EC are shown in Figures 3 and 4, respectively.

The analysis of the results from the bottles containing human VSMC and human vascular EC showed results similar to those obtained in Experiment 2, hence confirming the previously obtained results and the unexpected and surprising effects of the MYH11-siRNA on inhibition of VSMC proliferation without significant inhibition of EC proliferation.

### Experiment 4

After identifying and confirming the promising results arising from the MYH11 -siRNA gene silencer, our next step was to assess a likely class effect of this drug, that is, whether the promising results obtained with the gene silencer MYH11-siRNA may extend to other gene silencers of VMSC structural and functional proteins. To that end, Experiment 4 was carried out in human VSMC culture medium consisting of the following groups: lentivirus + gene silencer myosin heavy chain 11 (MYH11-siRNA), lentivirus + gene silencer myosin light chain 9 (MYL9-siRNA), lentivirus + gene silencer TRIO and F-actin binding protein (TRIOBP-siRNA), lentivirus + gene silencer calponin 1 (CNN1-siRNA), lentivirus + gene silencer myosin light chain kinase (MYLK-siRNA), lentivirus + gene silencer myosin phosphatise Rho interacting protein (MPRIP-siRNA), and full control (no vehicle and no gene silencer).

The MYH11-siRNA, MYL9-siRNA, TRIOBP-siRNA and CNN1-siRNA are gene silencers directed at VSMC structural proteins while the MYLK-siRNA and MPRIP-siRNA are gene silencers directed at VSMC functional proteins.

The doses of MYH11-siRNA, MYL9-siRNA, TRIOBP-siRNA, CNN1-siRNA, MYLK-siRNA, and MPRIP-siRNA were 5µL aliquots from 10 - 1000µM of siRNA in 100 µL/mL of transfection medium.

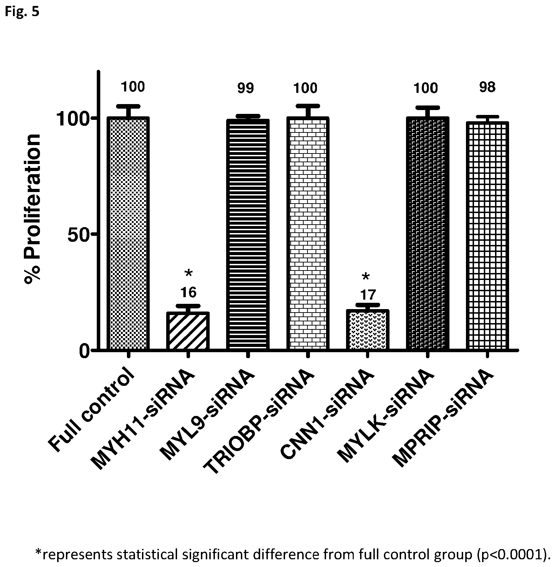

The results of the bottles containing human VSMC are shown in Figure 5. MYH11 -siRNA and CNN1-siRNA were the only ones among six specific gene silencers of structural and functional proteins studied to significantly inhibit VSMC proliferation.

The MYH11-siRNA and CNN1-siRNA gene silencers probably exert some blocking activity exclusively linked to the MYH11 and CNN1 genes. It is irrefutable that according to the results of Experiment 4 the MYH11-siRNA and CNN1-siRNAare the only gene silencers capable of significantly inhibiting VSMC proliferation without interfering with EC proliferation. Therefore, this blocking activity is not due to a class effect since the silencing of several other genes related to VSMC structural and functional proteins does not provide the same significant inhibition of VSMC proliferation.

Thus, both MYH11-siRNA and CNN1-siRNA exert, independently of each other, blocking activity on one or more proteins with a key role in formation and hyperproliferation of young VSMC. The exacerbated and disorganized proliferation of young VSMC in the media layer of the arterial vessels followed by their migration through the metallic stent struts or BVS struts towards the vessel lumen can lead to stent or BVS obstruction which is called ISR when greater than 50%.

### Experiment 5

The use of lentiviruses and plasmids for transfection of gene silencers is difficult to apply to the manufacturing of DES, BVS, DEB, and other devices which are often sterilized by processes that involve high temperatures such as ethylene oxide (ETO). Therefore, in this experiment, another method of carrying gene silencers into the vascular wall cells based on nanoparticles was tested.

Experiment 5 was carried out by using nanoparticles of 1, 2 distearoyl-sn-glycero-3-phosphoethanolamine-Nmethxy (polyethyleneglycol)-2000 (DSPE-PEG) as a drug and gene silencer carrying vehicle instead of lentiviruses and plasmids which were used in the previous experiments. That being so, 8 groups were included for both assessment of nanoparticles as a vehicle and final confirmation of the previous results: control (nanoparticles only), MYH11-siRNA in nanoparticles, CNN1-siRNA in nanoparticles, sirolimus in nanoparticles, paclitaxel in nanoparticles, TNFRSF11B-siRNA in nanoparticles, NCF4-siRNA in nanoparticles, and MYL9-siRNA+GAPDH in nanoparticles.

The groups of sirolimus in nanoparticles and paclitaxel in nanoparticles were included to compare the efficacy in inhibiting VSMC proliferation. The groups of NFRSF11B-siRNA in nanoparticles and NCF4-siRNA in nanoparticles were tested again with a different vehicle for final confirmation of the negative results. The group of MYL9-siRNA+GAPDH in nanoparticles was studied to evaluate the vehicle efficacy, that is, to ensure that gene silencers would enter VSMC and EC using nanoparticles as a vehicle. The MYL9-siRNA gene silencer was chosen as a gene silencer control because it does not have any inhibitory effect on both VSMC and EC proliferation according to the data obtained in Experiment 4. MYL9-siRNA gene silencer was marked with GAPDH for later verification of its transfection into the cells through fluorescence microscopy. Finally, pure nanoparticles were used as a vehicle control, that is, with neither drug nor gene silencer.

The nanoparticles with sirolimus and paclitaxel were prepared with DSPE-PEG-NH2 (1,2-distearoyl-sn-glycero-3-phosphoethanolamine-N[amino (polyethyleneglycol)-2000]. Both sirolimus and paclitaxel were solubilized in 1mL of acetone (organic phase) together with the PLGA polymer. The aqueous phase consisted of DSPE-PEG-NH2 solubilized in 10 mL of water Milli Q, under stirring and at a bath temperature of 40 C. Then the organic phase was injected into the aqueous phase using a 20G needle, the suspension being kept under moderate stirring for 5 minutes and then concentrated to a final volume of 10 mL in a rotatory evaporator to eliminate the organic solvent and adjust the final concentration of the drug. Sirolimus and paclitaxel concentrations ranging from 10µg/ml to 100µg/ml were tested.

The hybrid lipid-polymer nanoparticles loaded with gene silencers (siRNA) were prepared by the nanoprecipitation method. The aqueous phase consisted of the gene silencer solubilized in 10 mL of water Milli Q under stirring and at a bath temperature of 30oC. The organic phase containing a lipid cationic compound DSPE-PEG-NH2 (1, 2-distearoyl-sn-glycero-3phosphoethanolamine-N-[amino (polyethyleneglycol)-2000] and the PLGA polymer were then injected into the aqueous phase using a 20G needle, the suspension being kept under moderate stirring for 5 minutes and then concentrated to a final volume of 10 mL in a rotatory evaporator to eliminate the organic solvent and adjust the final concentration. Gene silencer concentrations ranging from 10µg/ml to 100µg/ml were tested.

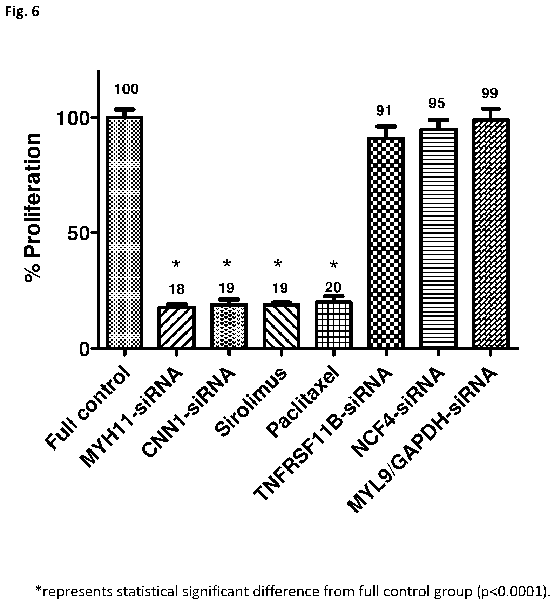

The results of the bottles containing human VSMC are shown in Figure 6. There was a significant inhibition of VSMC proliferation by the gene silencers MYH11 siRNA and CNN1-siRNA as well as by the sirolimus and paclitaxel antiproliferative drugs in comparison with the control, and no significant difference among the four groups themselves (two gene silencers and two antiproliferative drugs). The TNFRSF11B-siRNA and NCF4-siRNA gene silencers showed no significant difference in inhibition of VSMC proliferation as compared to the control. The set of results obtained in this experiment supports the previous results of the above unprecedented experiments that demonstrated the inefficacy of the TNFRSF11B-siRNA and NCF4-siRNA gene silencers in inhibiting VSMC hyperproliferation, but revealed the MYH11-siRNA and CNN1-siRNA gene silencers to be effective in inhibiting VSMC hyperproliferation. The lack of significant difference in inhibition of VSMC proliferation by the MYL9-siRNA gene silencer as compared to the control has already been expected since said gene silencer was used as a gene silencer control; however, when the MYL9-siRNA was marked with GAPDH and VSMC of this group was verified by fluorescence microscopy, it was found that the nanoparticles effectively entered into the VSMC.

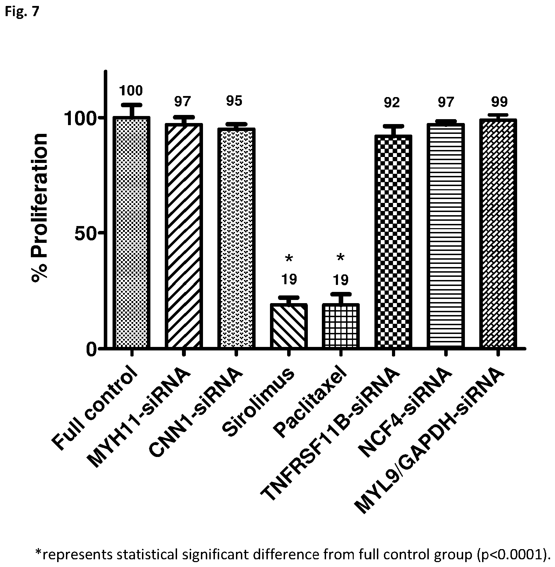

The results of the bottles containing human vascular endothelial cells are shown in Figure 7.

The results of the human EC culture were also similar to those of the previous experiments. There was no statistically significant difference in EC proliferation between the MYH11-siRNA, TNFRSF11B-siRNA, NCF4siRNA, and MYL9-siRNA gene silencers and the control group. Sirolimus and paclitaxel determined as already expected statistically significant inhibition of EC proliferation as compared to both the control group and all the gene silencers tested.

### Experiment 6

Experiment 6 was an in vivo study in swine comparing MYH11-siRNA-eluting stent and CNN1-siRNA-eluting stent with bare metal stent and sirolimus-eluting stent.

Eigtheen commercially available bare metal stents 2.75x15mm were covered with MYH11-siRNA in nanoparticles (9 stents) or CNN1-siRNA in nanoparticles (9 stents) using a spray nozzle machine to create an abluminal cover in order to deliver the gene silencers locally in the inner surface of the vessel.

The hybrid lipid-polymer nanoparticles loaded with gene silencers (siRNA) were prepared by the nanoprecipitation method. The aqueous phase consisted of the gene silencer solubilized in 10 mL of water Milli Q under stirring and at a bath temperature of 30oC. The organic phase containing a lipid-cationic compound DSPE-PEG-NH2 (1,2-distearoyl-sn-glycero-3-phosphoethanolamine-N-[amino(polyethyleneglycol)-2000] and the PLGA polymer was then injected into the aqueous phase using a 20G needle, the suspension being kept under moderate stirring for 5 minutes and then concentrated to a final volume of 10 mL in a rotatory evaporator to eliminate the organic solvent and adjust the final concentration. MYH11-siRNA and CNN1-siRNA concentrations ranging from 10µg/ml to 100µg/ml were tested.

After covering the abluminal surface of the 18 stents with MYH11 - siRNA in nanoparticles (9 stents) or CNN1-siRNA in nanoparticles (9 stents), the stents were left to dry during 60 minutes, packaged in DuPont Tyvek^{™} and then sterilized with ethylene oxide (EtO) at 50C.

Nine Large White swine were submitted to stent deployment in the coronary arteries, 1 stent being deployed in the left anterior descendent (LAD), 2 stents in the circumflex (CX) and 1 stent in the right coronary artery (RCA). The stents were deployed in a diameter ratio of 1.1 in relation to the coronary artery diameter, that is, with a mild oversize, in order to stimulate a similar inflammatory reaction that causes in-stent restenosis, to evaluate the efficacy of the gene silencers in controlling in-stent neointimal hyperplasia in a in vivo model. This oversized stent deployment protocol was previously validated (Lowe HC, Schwartz RS, Mac Neill BD, Jang IK, Hayase M, Rogers C, et al. The porcine coronary model of in-stent restenosis: Current status in the era of drug-eluting stents. Catheter CardiovascInterv. 2003:60:515-23.)

The prolonged time of stent re-endothelialization is the critical point of the current commercially available drug-eluting stents. So sirolimus and analogues as paclitaxel decrease the proliferation of vascular smooth muscle cells preventing in-stent neointimal hyperplasia, as well as suppress the proliferation of endothelial cells prolonging the time of stent re-endothelialization. Differently from sirolimus and its analogues and paclitaxel, the gene silencers have a selective action on decreasing the proliferation of smooth muscle cells without interfering with the endothelial cells proliferation. Therefore, in order to evaluate the time of stent re-endothelialization, angiographic and intravascular ultrasound follow-up were scheduled at 30, 90 and 180 days after stent deployment.

The intravascular ultrasound analysis was performed tracing 31 frames for each stent with 0.5mm apart each frame from the distal stent border to the proximal stent border. The data were analysed using one-way ANOVA with Bonferroni post hoc test.

The data of intravascular ultrasound in the index procedure as well as the results regarding in-stent neointimal proliferation at 30, 90 and 180 days after stent deployment are shown in table 1, while the comparative data of intravascular ultrasound regarding stent re-endothelialization at 30, 90 and 180 days after stent deployment are shown in table 2.

**Table 1: Comparative data from intravascular ultrasound at baseline and at 30, 90 and 180 days after stent deployment in swine.**

| | Bare Metal | Sirolimus | MYH11-siRNA | CNN1-siRNA | p |
|---|---|---|---|---|---|
| RD | 2.74±0.08mm | 2.73±0.08mm | 2.72±0.09mm | 2.73±0.08mm | p=0.9495 |
| MLD | | | | | |
| pre stent | 2.74±0.08mm | 2.73±0.08mm | 2.72±0.09mm | 2.73±0.08mm | p=0.9495 |
| post stent | 3.02±0.09mm | 3.01±0.10mm | 3.00±0.12mm | 3.00±0.11mm | p=9603 |
| follow-up | 2.11+0.12mm* | 2.68±0.13mm | 2.70±0.14mm | 2.73±0.13mm | p<0.0001 |
| LLL | 0.91±0.11mm* | 0.33±0.12mm | 0.30±0.14mm | 0.28±0.11mm | p<0.0001 |
| MLA | | | | | |
| post stent | 7.08±0.06mm² | 7.06±0.07mm² | 7.06±0.06mm² | 7.07±0.07mm² | p=0.8546 |
| follow-up | 4.67±0.23mm² | 6.21±0.16mm² | 6.35±0.14mm² | 6.43±0.13mm² | p<0.0001 **(a,b)** |
| | **a** | **b** | **be** | **c** | p= 0.03 **(b,c)** |
| Obstruction | | | | | p<0.0001 |
| 30 days | 32.3±2.4%* | 11.4±2.2% | 10.5±2.8% | 9.6±2.3% | p<0.0001 |
| 90 days | 33.4±2.2%* | 12.3±2.1% | 11.4±2.7% | 10.4±2.5% | p<0.0001**(a,b)** |
| 180 days | 35.1±2.8% **a** | 13.8±2.4% **b** | 12.4±2.1% **be** | 10.9±2.6% **c** | p= 0.012 **(b,c)** |

| | | | | | |
|---|---|---|---|---|---|
| - RD: reference diameter of the vessel; MLD: minimal luminal diameter; LLL: late lumen loss; MLA: minimal luminal area; obstruction: average of percentual obstruction in the whole stent length; ^{∗}: p significant; letters a,b,c: different p values. | | | | | |

All three drug-eluting stents, that is, sirolimus-eluting stent, MYH11-siRNA-eluting stent and CNN1-siRNA-eluting stent, showed higher minimal luminal area and lower obstruction at 30, 90 and 180 days in comparison with the bare metal stent. There was no statistically significant difference between sirolimus-eluting stent, MYH11-siRNA-eluting stent and CNN1-siRNA-eluting stent.

The above findings show that both MYH11-siRNA-eluting stent and CNN1-siRNA-eluting stent are superior to bare metal stent and as effective as sirolimus-eluting stent in promoting inhibition of in-stent neointimal hyperplasia. Therefore, the gene silencers MYH11-siRNA and CNN1-siRNA are able to suppress the proliferation of VSMC and consequently in-stent neointimal hyperplasia through a novel mechanism of action by blocking the expression of specific and pivotal genes involved in the proliferation of VSMC, which is completely different from the mechanism of action of cell antiproliferative drugs, such as sirolimus and its analogues and paclitaxel, which act at the checkpoints of the cell cycle. Therefore, sirolimus and its analogues act in the G1-S phase of the cell cycle while paclitaxel acts in the G2-M phase of the cell cycle, both group of drugs stopping the cell cycle of any kind of cell (including VSMC and EC) and inhibiting cell division and posterior cell proliferation.

The data regarding stent re-endothelialization in table 2 show important results.

**Table 2: Comparative data regarding re-endothelialization from intravascular ultrasound at 30, 90 and 180 days after stent deployment in swine.**

| | Bare Metal | Sirolimus | MYH11-siRNA | CNN1-siRNA | p |
|---|---|---|---|---|---|
| 30 days | 98±3.1% | 48±3.3%* | 97±3.1% | 98±3.0% | p<0.0001 |
| 90 days | 100±2.9% | 89±3.8%* | 100±2.8% | 100±2.7% | p<0.001 |
| 180 days | 100% | 100% | 100% | 100% | - |

At 30 days, the intravascular ultrasound analysis revealed a similar percentage of stent struts coverage in the 31 stent frames analyzed per stent in the bare metal stent, MYH11-siRNA-eluting stent and CNN1-siRNA-eluting stent groups, whereas the sirolimus-eluting stent group showed a statistically significant difference of stent struts coverage in comparison with the bare metal stent, MYH11-siRNA-eluting stent and CNN1-siRNA-eluting stent groups.

The intravascular ultrasound results at 90 days showed complete stent strut coverage in the bare metal stent, MYH11-siRNA-eluting stent and CNN1-siRNA-eluting stent groups, whereas the sirolimus-eluting stent group evidenced only partial stent strut coverage with a statistically significant difference in comparison with the bare metal stent, MYH11-siRNA-eluting stent and CNN1-siRNA-eluting stent groups.

At 180 days, the intravascular ultrasound results evidenced complete stent strut coverage in all of the 4 groups.

The time of stent strut full coverage is the current challenge regarding drug-eluting stents because stent strut coverage is directly related to stent thrombosis and, consequently, to duration of dual antiplatelet therapy. Therefore, a drug-eluting stent that inhibits VSMC and neointimal hyperplasia as sirolimus-eluting stent and also promotes rapid re-endothelialization as a bare metal stent is required. Experiment 6 showed that both MYH11-siRNA-eluting stent and CNN1-siRNA-eluting stent were able to promote sustained inhibition of in-stent hyperplasia as sirolimus-eluting stent but also allowed rapid re-endothelialization as a bare metal stent by means of a novel mechanism of action never before described in the medical literature.

### Experiment 7

In Experiment 7, we evaluated other 32 gene silencers related to structural and functional proteins of human VSMC which had not been previously tested or even mentioned in the above experiments.

The gene silencers were prepared in nanoparticles of 1,2-distearoyl-sn-glycero-3-phosphoethanolamine-N-methxy(polyethyleneglycol)-2000 (DSPE-PEG) as a vehicle for the gene silencers by using the same method as in Experiment 5.

Four groups (1) control (nanoparticles only), (2) sirolimus in nanoparticles, (3) MYH11-siRNA in nanoparticles and (4) CNN1-siRNA in nanoparticles were used to compare the efficacy in inhibiting VSMC proliferation.

The nanoparticles with sirolimus were prepared with DSPE-PEG-NH2 (1,2-distearoyl-sn-glycero-3-phosphoethanolamine-N-[amino(polyethyleneglycol)-2000]. Sirolimus was solubilized in 1 mL of acetone (organic phase) together with the PLGA polymer. The aqueous phase consisted of DSPE-PEG-NH2 solubilized in 10 mL of water Milli Q, under stirring and at a bath temperature of 40°C. Next, the organic phase was injected into the aqueous phase using a 20G needle, the suspension being kept under moderate stirring for 5 minutes and then concentrated to a final volume of 10 mL in a rotatory evaporator to eliminate the organic solvent and adjust the final concentration of the drug. Sirolimus concentration ranging from 10µg/ml to 100µg/ml was tested.

The hybrid lipid-polymer nanoparticles loaded with gene silencers (siRNA) were prepared by the nanoprecipitation method. The aqueous phase consisted of the gene silencer solubilized in 10 mL of water Milli Q under stirring and at a bath temperature of 30oC. The organic phase containing a lipid-cationic compound DSPE-PEG-NH2 (1,2-distearoyl-sn-glycero-3-phosphoethanolamine-N-[amino(polyethyleneglycol)-2000] and the PLGA polymer was then injected into the aqueous phase using a 20G needle, the suspension being kept under moderate stirring for 5 minutes and then concentrated to a final volume of 10 mL in a rotatory evaporator to eliminate the organic solvent and adjust the final concentration. Gene silencer concentrations ranging from 10µg/ml to 100µg/ml were tested.

Efficacy in significantly inhibiting the proliferation of human VSMC was defined as ≤50% of human VSMC proliferation in the bottles containing these specific cells, whereas a nonsignificant effect on the inhibition of human EC was defined as ≥90% of human EC proliferation in the bottles containing human EC.

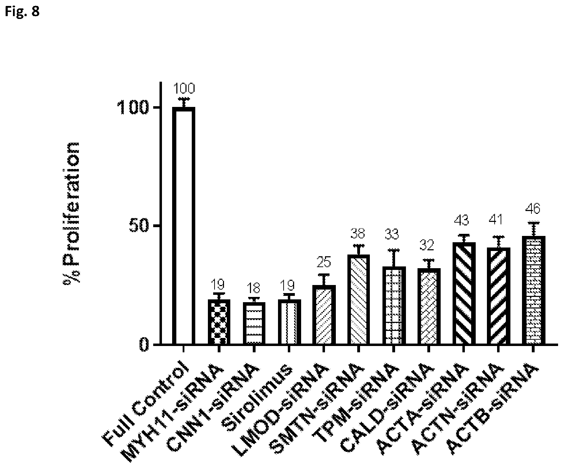

The results of the bottles containing human VSMC revealed that out of the additional 32 gene silencers tested only 7 genes silencers, that is leiomodin 1 (LMOD)-siRNA, smoothelin (SMTN)-siRNA, tropomyosin (TPM)-siRNA, caldesmon 1 (CALD)-siRNA, actinin (ACTN)-siRNA, actin alpha (ACTA)-siRNA, and actin beta (ACTB)-siRNA, promoted significant inhibition of human VSMC proliferation according to the criteria above mentioned, that is, ≤50% of human VSMC proliferation in the bottles containing these specific cells, as shown in Figure 8.

The gene silencers MYH11-siRNA and CNN1-siRNA were the strongest inhibitors of human VSMC since they were the only ones to promote an inhibition rate higher than 80% (19% and 18% of human VSMC proliferation, respectively) which was similar to sirolimus (19% of human VSMC proliferation). The LMOD-siRNA was the only gene silencer to promote a human VSMC inhibition rate ranging between 70 and 80% (25% of human VSMC proliferation), which was close but not so strong as sirolimus, MYH11-siRNA and CNN1-siRNA. Next, at a human VSMC inhibition rate ranging between 60 and 70%, this experiment evidenced SMTN-siRNA, TPM-siRNA, and CALD-siRNA, which showed 38%, 33%, and 32% of human VSMC proliferation, respectively. Finally, ACTN-siRNA, ACTA-siRNA, and ACTB-siRNA demonstrated a human VSMC inhibition rate ranging between 50 and 60% through 41%, 43%, and 46% of human VSMC proliferation, respectively.

The set of results obtained in this experiment support again the previous results of these series of unprecedented experiments that revealed MYH11-siRNA and CNN1-siRNA gene silencers to be as effective as sirolimus in inhibiting VSMC hyperproliferation; moreover they also demonstrated the efficacy of other 7 gene silencers - LMOD-siRNA, SMTN-siRNA, TPM-siRNA, CALD-siRNA, ACTN-siRNA, ACTA-siRNA, and ACTB-siRNA - in significantly inhibiting human VSMC proliferation. Therefore, a total of 9 gene silencers were able to significantly inhibit human VSMC hyperproliferation; however, they exhibited different potency or effectiveness.

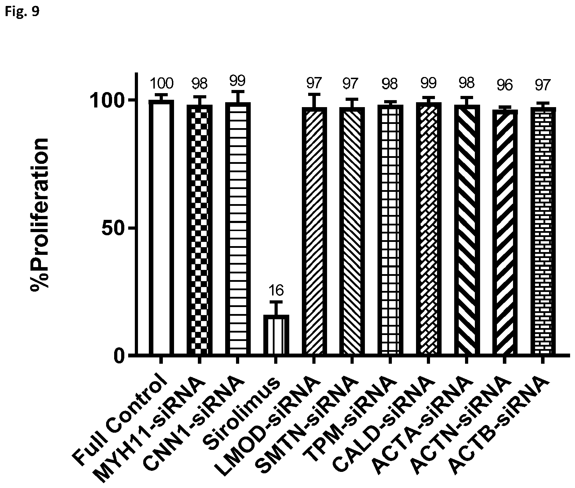

Experiment 7 also evaluated the effect of the 9 gene silencers MYH11-siRNA, CNN1-siRNA, LMOD-siRNA, SMTN-siRNA, TPM-siRNA, CALD-siRNA, ACTN-siRNA, ACTA-siRNA, and ACTB-siRNA, as well as nanoparticles only (control) and sirolimus in inhibiting human EC proliferation. The results of the bottles containing human vascular endothelial cells are shown in Figure 9. There was no statistically significant difference in EC proliferation among all the 9 gene silencers (MYH11-siRNA, CNN1-siRNA, LMOD-siRNA, SMTN-siRNA, TPM-siRNA, CALD-siRNA, ACTN-siRNA, ACTA-siRNA, and ACTB-siRNA) tested themselves as well as compared to the control group composed of nanoparticles only (neither drug nor gene silencer). As already described in the literature and observed in the above experiments, sirolimus determined statistically significant inhibition of EC proliferation as compared to both the control group and all the 9 gene silencers tested. Thus, all the 9 gene silencers that effectively inhibited the proliferation of human VSMC did not interfere with the proliferation of human EC, which make them potential candidates for a gene silencer-eluting medical device since they are able to control human VSMC proliferation without suppressing human EC proliferation, hence allowing rapid vessel and/or medical device re-endothelialization.

### Experiment 8

The previous 7 experiments allowed to identify 9 gene silencers related to structural and functional proteins of human VSMC not previously studied or tested as agents able to control human VSMC proliferation without suppressing human EC proliferation. These experiments also revealed that said 9 gene silencers have different potency or efficacy in inhibiting human VSMC when using the same doses.

Therefore, aiming to clarify the relationship between gene silencer dosage and potency/efficacy in inhibiting human VSMC, we performed Experiment 8 which evaluated 3 different doses of 5 gene silencers tested in Experiment 7. That being so, we selected CNN1-siRNA and MYH11-siRNA which were the most effective gene silencers; LMOD-siRNA that promoted an inhibitory effect on human VSMC in a range between 70 and 80%; SMTN-siRNA which caused an inhibitory effect on human VSMC in a range between 60 and 70% and ACTB-siRNA that showed the weakest response in human VSMC inhibition among the 9 gene silencers considered effective. The 5 selected gene silencers were compared with nanoparticles only (control) and sirolimus.

In one embodiment, three different escalating doses of gene silencers were tested:
a) Standard dose, 8 microliters per well of the standard gene silencer solution concentration;
b) High dose, 24 microliters per well of the standard gene silencer solution concentration; and
c) Very high dose, 24 microliters per well of a gene silencer solution with double concentration in comparison with the standard gene silencer solution.

The standard gene silencer solution was prepared using the same methodology of the previous experiments, that is, by the nanoprecipitation method. The aqueous phase consisted of the gene silencer solubilized in 10 mL of water Milli Q under stirring and at a bath temperature of 30oC. The organic phase containing a lipid-cationic compound DSPE-PEG-NH2 (1, 2-distearoyl-sn-glycero-3-phosphoethanolamine-N-[amino (polyethyleneglycol)-2000] and the PLGA polymer was then injected into the aqueous phase using a 20G needle, the suspension being kept under moderate stirring for 5 minutes and then concentrated to a final volume of 10 mL in a rotatory evaporator to eliminate the organic solvent and adjust the final concentration. Gene silencer concentrations ranging from 10µg/ml to 100µg/ml were tested.

The double concentration gene silencer solution was also prepared using the nanoprecipitation method. The aqueous phase consisted of the gene silencer solubilized in 10 mL of water Milli Q under stirring and at a bath temperature of 30oC. The organic phase containing a lipid-cationic compound DSPE-PEG-NH2 (1, 2-distearoyl-sn-glycero-3-phosphoethanolamine-N-[amino (polyethyleneglycol)-2000] and the PLGA polymer was then injected into the aqueous phase using a 20G needle, the suspension being kept under moderate stirring for 10 minutes and then concentrated to a final volume of 5 mL in a rotatory evaporator to eliminate the organic solvent and adjust the final concentration. Gene silencer concentrations ranging from 20µg/ml to 200µg/ml were tested.

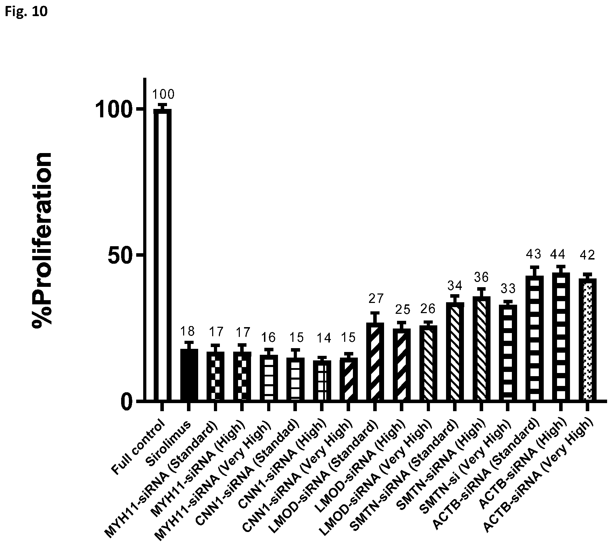

The results of the bottles containing human vascular endothelial cells are shown in Figure 10.

There was no statistically significant difference among the 3 different escalating doses tested for each of the 5 gene silencers evaluated. Therefore, similarly to the sirolimus effect on the human VSMC proliferation, the gene silencers also have a wide range of doses at which the agent is effective and its effectiveness is not dose-dependent. That being so, there is probably a threshold in this wide range of doses at which the maximum effect is reached (minimum effective dosage). Furthermore, once the minimum effective dosage is reached the VSMC receptors saturate, which makes the effect non-dose-dependent from said minimum effective dosage threshold.

All 5 gene silencers evaluated as well as sirolimus showed the same magnitude of human VSMC inhibition in comparison with the control group as in the previous experiments of this patent application hereof. Therefore, Experiment 8 supports the stability of the effect of gene silencers on inhibiting human VSMC proliferation as well as the reproducibility of the methodology used for the experiments.

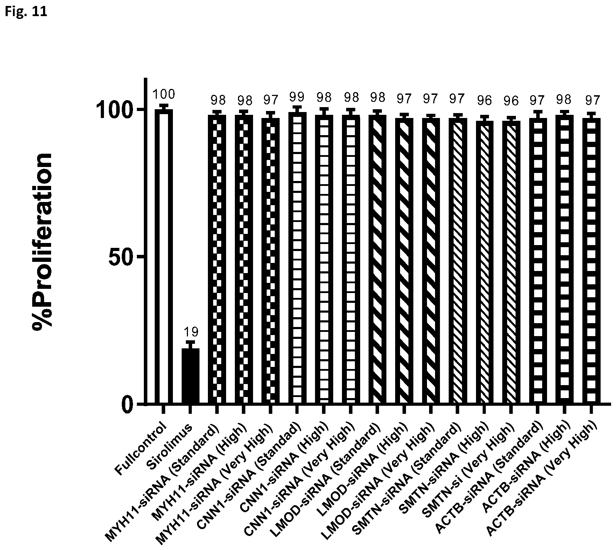

Furthermore, aiming to evaluate the effect of gene silencers escalating doses on human EC, we tested 3 different escalating doses of 5 gene silencers CNN1-siRNA, MYH11-siRNA, LMOD-siRNA, SMTN-siRNA and ACTB-siRNA in comparison with nanoparticles only (control) and sirolimus in bottles containing human EC. The results of the bottles containing human EC cells are shown in Figure 11.

There was no statistically significant difference among the 3 different escalating doses tested for each of the 5 gene silencers evaluated themselves, as well as neither of the 3 different escalating doses tested for each of the 5 gene silencers evaluated differed significantly in comparison with the control. As already expected, sirolimus showed statistically significant difference in comparison with the control as well as with all the 3 different escalating doses tested for each of the 5 gene silencers evaluated. Therefore, even high or very high doses of gene silencers do not interfere with the proliferation of human EC.

## Claims

1. A device to promote rapid strut coverage and vascular endothelial coverage, the device comprising:
a) an eluting medical device; and
b) at least one gene silencer (siRNA)
- wherein the eluting medical device is coated or filled in with at least one gene silencer;
- wherein the gene silencer is selected from myosin heavy chain 11 (MYH11)-siRNA, calponin 1 (CNN1)-siRNA, leiomodin 1 (LMOD)-siRNA, smoothelin (SMTN)-siRNA, tropomyosin (TPM)-siRNA, caldesmon 1 (CALD)-siRNA, actinin (ACTN)-siRNA, actin alpha (ACTA)-siRNA, and actin beta (ACTB)-siRNA;
- wherein the gene silencer is delivered locally in the blood vessel to enter the vessel wall and vascular smooth muscle cells (VSMC) for the selective inhibition of the VSMC hyperproliferation without inhibiting endothelial cells (EC) proliferation.

2. The device as claimed in claim 1, wherein the device comprises a gene silencer vehicle, said gene silencer vehicle facilitating elution of the gene silencer and allowing the gene silencer to enter the blood vessel wall and VSMC.

3. The device as claimed in claim 1, wherein said eluting medical device is selected from any implantable device and non-implantable local drug delivery device.

4. The device as claimed in claim 3, wherein the implantable device is selected from a metallic stent and a bioresorbable vascular scaffold (BVS).

5. The device as claimed in claim 3, wherein said non-implantable local drug delivery device is selected from drug eluting balloon (DEB), local drug delivery catheter and any other medical device capable of locally delivering MYH11-siRNA, CNN1-siRNA, LMOD-siRNA, SMTN-siRNA, TPM-siRNA, CALD-siRNA, ACTN-siRNA, ACTA-siRNA, and ACTB-siRNA.

6. The device as claimed in claim 2, wherein said gene silencer vehicle is chosen from a group consisting of lentiviruses and plasmids, nanoparticles, fluoropolymers, bioabsorbable polymers, non-absorbable polymers, or can even be polymer free (without any kind of polymer).

7. The device as claimed in claim 1, wherein said gene silencer is delivered alone or in combination with another gene silencer or with cell antiproliferative drugs.

8. The device as claimed in claim 1, wherein the gene silencer concentration ranges from 1 µg/ml to 1000 µg/ml, which is a wide concentration range, since the gene silencer can be spread over the medical device surface from a single very thin layer up to several thick layers to reach the desired amount of gene silencer to be locally delivered.

9. The device as claimed in claim 1, wherein the device promotes both permanent patency of the blood vessel and early re-endothelialization of a treated blood vessel segment.

10. An eluting medical device for use in a method for promoting rapid strut coverage and vascular endothelial coverage, the method comprising:
a) providing an eluting medical device and at least one gene silencer;
b) coating or filling in the eluting medical device with at least one gene silencer;
c) delivering the gene silencer to a target segment of blood vessel; and
d) selecting the gene silencer from the following gene silencers: myosin heavy chain 11 (MYH11)-siRNA, calponin 1 (CNN1)-siRNA, leiomodin 1 (LMOD)-siRNA, smoothelin (SMTN)-siRNA, tropomyosin (TPM)-siRNA, caldesmon 1 (CALD)-siRNA, actinin (ACTN)-siRNA, actin alpha (ACTA)-siRNA and actin beta (ACTB)-siRNA:
- wherein said gene silencer is delivered to the target segment of blood vessel to enter the vessel wall and VSMC for the selective inhibition of the VSMC hyperproliferation;
- wherein said gene silencer does not significantly interfere with EC production or can even stimulate it.

11. The eluting medical device for use in the method as claimed in claim 10, wherein the method further comprises loading said gene silencer in a gene silencer vehicle:
- wherein said gene silencer vehicle facilitates elution of the gene silencer;
- wherein said gene silencer vehicle allows the gene silencer to enter the target blood vessel wall and VSMC.

12. The eluting medical device for use in the method as claimed in claim 10, wherein the eluting medical device is selected from any implantable device and non-implantable local drug delivery device.

13. The eluting medical device for use in the method as claimed in claim 12, wherein the implantable device is selected from a metallic stent and a bioresorbable vascular scaffold (BVS).

14. The eluting medical device for use in the method as claimed in claim 12, wherein said non-implantable local drug delivery device is selected from a drug eluting balloon (DEB), a local drug delivery catheter and any other medical device capable of locally delivering MYH11-siRNA, CNN1-siRNA, LMOD-siRNA, SMTN-siRNA, TPM-siRNA, CALD-siRNA, ACTN-siRNA, ACTA-siRNA, and ACTB-siRNA.

15. The eluting medical device for use in the method as claimed in claim 11, wherein said gene silencer vehicle is chosen from a group consisting of lentiviruses and plasmids, nanoparticles, fluoropolymers, bioabsorbable polymers, non-absorbable polymers, or can even be polymer free (without any kind of polymer).

16. The eluting medical device for use in the method as claimed in claim 10, wherein said gene silencer is delivered alone or in combination with another gene silencer or with cell antiproliferative drugs.

17. The eluting medical device for use in the method as claimed in claim 10, wherein the gene silencer concentration ranges from 1 µg/ml to 1000 µg/ml, which is a wide concentration range, since the gene silencer can be spread over the medical device surface from a single very thin layer up to several thick layers to reach the desired amount of gene silencer to be locally delivered.

18. The eluting medical device for use in the method as claimed in claim 10, wherein the method promotes both permanent patency of the blood vessel and early re-endothelialization of a treated blood vessel segment.

## Patentansprüche

1. Vorrichtung zur Förderung einer schnellen Strebenabdeckung und der vaskulären Endothelabdeckung, wobei die Vorrichtung Folgendes umfasst:
a) eine eluierende medizinische Vorrichtung; und
b) mindestens einen Gen-Silencer (siRNA)
- wobei die eluierende medizinische Vorrichtung mit mindestens einem Gen-Silencer beschichtet oder gefüllt ist;
- wobei der Gen-Silencer ausgewählt ist aus Myosin schwere Kette 11 (MYH11)-siRNA, Calponin 1 (CNN1)-siRNA, Leiomodin 1 (LMOD)-siRNA, Smoothelin (SMTN)-siRNA, Tropomyosin (TPM)-siRNA, Caldesmon 1 (CALD)-siRNA, Actinin (ACTN)-siRNA, Actin alpha (ACTA)-siRNA und Actin beta (ACTB)-siRNA;
- wobei der Gen-Silencer lokal in das Blutgefäß abgegeben wird, um in die Gefäßwand und die vaskulären glatten Muskelzellen (VSMC) einzudringen, um die Hyperproliferation der VSMC selektiv zu hemmen, ohne die Proliferation der Endothelzellen (EC) zu hemmen.

2. Vorrichtung nach Anspruch 1, wobei die Vorrichtung ein Gen-Silencer-Vehikel umfasst, wobei das Gen-Silencer-Vehikel die Elution des Gen-Silencers erleichtert und es dem Gen-Silencer erlaubt, in die Blutgefäßwand und VSMC einzudringen.

3. Vorrichtung nach Anspruch 1, wobei die eluierende medizinische Vorrichtung ausgewählt ist aus einer beliebigen implantierbaren Vorrichtung und einer nicht-implantierbaren lokalen Arzneimittelabgabevorrichtung.

4. Vorrichtung nach Anspruch 3, wobei die implantierbare Vorrichtung ausgewählt ist aus einem metallischen Stent und einem bioresorbierbaren Gefäßgerüst (BVS).

5. Vorrichtung nach Anspruch 3, wobei die nicht-implantierbare Vorrichtung zur lokalen Medikamentenabgabe ausgewählt ist aus dem Medikamentenfreisetzungsballon (DEB), dem Katheter zur lokalen Medikamentenabgabe und jeder anderen medizinischen Vorrichtung, die in der Lage ist, MYH11-siRNA, CNN1-siRNA, LMOD-siRNA, SMTN-siRNA, TPM-siRNA, CALD-siRNA, ACTN-siRNA, ACTA-siRNA und ACTB-siRNA lokal abzugeben.

6. Vorrichtung nach Anspruch 2, wobei das Gen-Silencer-Vehikel ausgewählt ist aus der Gruppe bestehend aus Lentiviren und Plasmiden, Nanopartikeln, Fluorpolymeren, bioresorbierbaren Polymeren, nicht resorbierbaren Polymeren oder sogar polymerfrei (ohne jegliche Art von Polymer) sein kann.

7. Vorrichtung nach Anspruch 1, wobei der Gen-Silencer allein oder in Kombination mit einem anderen Gen-Silencer oder mit zellproliferationshemmenden Medikamenten verabreicht wird.

8. Vorrichtung nach Anspruch 1, wobei die Konzentration des Gen-Silencers im Bereich von 1 µg/ml bis 1000 µg/ml liegt, was ein breiter Konzentrationsbereich ist, da der Gen-Silencer von einer einzigen sehr dünnen Schicht bis zu mehreren dicken Schichten auf der Oberfläche der medizinischen Vorrichtung verteilt werden kann, um die gewünschte Menge des lokal abzugebenden Gen-Silencers zu erreichen.

9. Vorrichtung nach Anspruch 1, wobei die Vorrichtung sowohl die dauerhafte Durchgängigkeit des Blutgefäßes als auch die frühe Reendothelialisierung eines behandelten Blutgefäßsegments fördert.

10. Eluierende medizinische Vorrichtung zur Verwendung in einem Verfahren zur Förderung einer raschen Strebenabdeckung und vaskulären Endothelabdeckung, wobei das Verfahren Folgendes umfasst:
a) Bereitstellen einer eluierenden medizinischen Vorrichtung und mindestens eines Gen-Silencers;
b) Beschichten oder Füllen der eluierenden medizinischen Vorrichtung mit mindestens einem Gen-Silencer;
c) Verabreichen des Gen-Silencers an ein Zielsegment eines Blutgefäßes; und
d) Auswählen des Gen-Silencers aus den folgenden Gen-Silencern: Myosin schwere Kette 11 (MYH11)-siRNA, Calponin 1 (CNN1)-siRNA, Leiomodin 1 (LMOD)-siRNA, Smoothelin (SMTN)-siRNA, Tropomyosin (TPM)-siRNA, Caldesmon 1 (CALD)-siRNA, Actinin (ACTN)-siRNA, Actin alpha (ACTA)-siRNA und Actin beta (ACTB)-siRNA:
- wobei der Gen-Silencer an das Zielsegment des Blutgefäßes abgegeben wird, um in die Gefäßwand und die VSMC einzudringen und die Hyperproliferation der VSMC selektiv zu hemmen;
- wobei der Gen-Silencer die EC-Produktion nicht signifikant beeinträchtigt oder sie sogar stimulieren kann.

11. Eluierende medizinische Vorrichtung zur Verwendung in dem Verfahren nach Anspruch 10, wobei das Verfahren ferner Laden des Gen-Silencers in ein Gen-Silencer-Vehikel umfasst:
- wobei das Gen-Silencer-Vehikel die Elution des Gen-Silencers erleichtert;
- wobei das Gen-Silencer-Vehikel es dem Gen-Silencer erlaubt, in die Ziel-Blutgefäßwand und VSMC einzudringen.

12. Eluierende medizinische Vorrichtung zur Verwendung in dem Verfahren nach Anspruch 10, wobei die eluierende medizinische Vorrichtung aus einer beliebigen implantierbaren Vorrichtung und einer nicht-implantierbaren lokalen Arzneimittelabgabevorrichtung ausgewählt ist.

13. Eluierende medizinische Vorrichtung zur Verwendung in dem Verfahren nach Anspruch 12, wobei die implantierbare Vorrichtung ausgewählt ist aus einem metallischen Stent und einem bioresorbierbaren Gefäßgerüst (BVS).

14. Eluierende medizinische Vorrichtung zur Verwendung in dem Verfahren nach Anspruch 12, wobei die nicht-implantierbare Vorrichtung zur lokalen Medikamentenabgabe ausgewählt ist aus einem Medikamentenfreisetzungsballon (DEB), einem Katheter zur lokalen Medikamentenabgabe und jeder anderen medizinischen Vorrichtung, die in der Lage ist, MYH11-siRNA, CNN1-siRNA, LMOD-siRNA, SMTN-siRNA, TPM-siRNA, CALD-siRNA, ACTN-siRNA, ACTA-siRNA und ACTB-siRNA lokal abzugeben.

15. Eluierende medizinische Vorrichtung zur Verwendung in dem Verfahren nach Anspruch 11, wobei das Gen-Silencer-Vehikel ausgewählt ist aus der Gruppe bestehend aus Lentiviren und Plasmiden, Nanopartikeln, Fluorpolymeren, bioresorbierbaren Polymeren, nicht resorbierbaren Polymeren oder sogar polymerfrei (ohne jegliche Art von Polymer) sein kann.

16. Eluierende medizinische Vorrichtung zur Verwendung in dem Verfahren nach Anspruch 10, wobei der Gen-Silencer allein oder in Kombination mit einem anderen Gen-Silencer oder mit zellproliferationshemmenden Medikamenten verabreicht wird.

17. Eluierende medizinische Vorrichtung zur Verwendung in dem Verfahren nach Anspruch 10, wobei die Konzentration des Gen-Silencers im Bereich von 1 µg/ml bis 1000 µg/ml liegt, was ein breiter Konzentrationsbereich ist, da der Gen-Silencer von einer einzigen sehr dünnen Schicht bis zu mehreren dicken Schichten auf der Oberfläche der medizinischen Vorrichtung verteilt werden kann, um die gewünschte Menge des lokal abzugebenden Gen-Silencers zu erreichen.

18. Eluierende medizinische Vorrichtung zur Verwendung in dem Verfahren nach Anspruch 10, wobei das Verfahren sowohl die dauerhafte Durchgängigkeit des Blutgefäßes als auch die frühe Reendothelialisierung eines behandelten Blutgefäßsegments fördert.

## Revendications

1. Dispositif destiné à favoriser une couverture de support et une couverture endothéliale vasculaire rapides, le dispositif comprenant :
a) un dispositif médical d'élution ; et
b) au moins un inactivateur de gène (pARNi)
- dans lequel le dispositif médical d'élution est revêtu ou rempli d'au moins un inactivateur de gène ;
- dans lequel l'inactivateur de gène est choisi parmi la chaîne lourde 11 de la myosine (MYH11)-pARNi, la calponine 1 (CNN1)-pARNi, la léiomodine 1 (LMOD)-pARNi, la smootheline (SMTN)-pARNi, la tropomyosine (TPM)-pARNi, le caldesmon 1 (CALD)-pARNi, l'actinine (ACTN)-pARNi, l'actine alpha (ACTA)-pARNi et l'actine bêta (ACTB)-pARNi ;
- dans lequel l'inactivateur de gène est administré localement dans le vaisseau sanguin pour pénétrer dans la paroi de vaisseau et les cellules musculaires lisses vasculaires (CMLV) pour l'inhibition sélective de l'hyperprolifération des CMLV sans inhiber la prolifération des cellules endothéliales (CE).

2. Dispositif selon la revendication 1, dans lequel le dispositif comprend un vecteur d'inactivateur de gène, ledit vecteur d'inactivateur de gène facilitant l'élution de l'inactivateur de gène et permettant à l'inactivateur de gène de pénétrer dans la paroi de vaisseau sanguin et les CMLV.

3. Dispositif selon la revendication 1, dans lequel ledit dispositif médical d'élution est choisi parmi n'importe quel dispositif implantable et dispositif d'administration locale de médicament non implantable.

4. Dispositif selon la revendication 3, dans lequel le dispositif implantable est sélectionné parmi un stent métallique et un échafaudage vasculaire biorésorbable (BVS).

5. Dispositif selon la revendication 3, dans lequel ledit dispositif d'administration locale de médicament non implantable est sélectionné parmi un ballon d'élution de médicament (DEB), un cathéter d'administration locale de médicament et tout autre dispositif médical capable d'administrer localement MYH11-pARNi, CNN1-pARNi, LMOD-pARNi, SMTN-pARNi, TPM-pARNi, CALD-pARNi, ACTN-pARNi, ACTA-pARNi et ACTB-pARNi.

6. Dispositif selon la revendication 2, dans lequel ledit vecteur d'inactivateur de gène est choisi parmi un groupe constitué de lentivirus et de plasmides, de nanoparticules, de fluoropolymères, de polymères biorésorbables, de polymères non résorbables, voire peut être sans polymère (sans aucun type de polymère).

7. Dispositif selon la revendication 1, dans lequel ledit inactivateur de gène est administré seul ou en combinaison avec un autre inactivateur de gène ou avec des médicaments antiprolifératifs cellulaires.

8. Dispositif selon la revendication 1, dans lequel la concentration d'inactivateur de gène est comprise entre 1 µg/ml et 1000 µg/ml, ce qui est une large plage de concentration, puisque l'inactivateur de gène peut être étalé sur la surface du dispositif médical d'une seule couche très mince jusqu'à plusieurs couches épaisses pour atteindre la quantité souhaitée d'inactivateur de gène à administrer localement.

9. Dispositif selon la revendication 1, dans lequel le dispositif favorise à la fois la perméabilité permanente du vaisseau sanguin et la ré-endothélialisation précoce d'un segment de vaisseau sanguin traité.

10. Dispositif médical d'élution destiné à être utilisé dans un procédé destiné à favoriser une couverture de support et une couverture endothéliale vasculaire rapides, le procédé comprenant :
a) une fourniture d'un dispositif médical d'élution et d'au moins un inactivateur de gène ;
b) un revêtement ou un remplissage du dispositif médical d'élution avec au moins un inactivateur de gène ;
c) une administration de l'inactivateur de gène à un segment cible de vaisseau sanguin ; et
d) une sélection de l'inactivateur de gène parmi les inactivateurs de gène suivants : chaîne lourde 11 de la myosine (MYH11)-pARNi, calponine 1 (CNN1)pARNi, léiomodine 1 (LMOD)-pARNi, smootheline (SMTN)-pARNi, tropomyosine (TPM)-pARNi, caldesmon 1 (CALD)-pARNi, actinine (ACTN)-pARNi, actine alpha (ACTA)-pARNi et actine bêta (ACTB)-pARNi :
- dans lequel ledit inactivateur de gène est administré au segment cible de vaisseau sanguin pour pénétrer dans la paroi de vaisseau et les CMLV pour l'inhibition sélective de l'hyperprolifération des CMLV ;
- dans lequel ledit inactivateur de gène n'interfère pas significativement avec la production de CE ou peut même la stimuler.

11. Dispositif médical d'élution destiné à être utilisé dans le procédé selon la revendication 10, dans lequel le procédé comprend en outre le chargement dudit inactivateur de gène dans un vecteur d'inactivateur de gène :
- dans lequel ledit vecteur d'inactivateur de gène facilite l'élution de l'inactivateur de gène;
- dans lequel ledit vecteur d'inactivateur de gène permet à l'inactivateur de gène de pénétrer dans la paroi de vaisseau sanguin cible et dans les CMLV.

12. Dispositif médical d'élution destiné à être utilisé dans le procédé selon la revendication 10, dans lequel le dispositif médical d'élution est sélectionné parmi n'importe quel dispositif implantable et dispositif d'administration locale de médicament non implantable.

13. Dispositif médical d'élution destiné à être utilisé dans le procédé selon la revendication 12, dans lequel le dispositif implantable est sélectionné parmi un stent métallique et un échafaudage vasculaire biorésorbable (BVS).

14. Dispositif médical d'élution destiné à être utilisé dans le procédé selon la revendication 12, dans lequel ledit dispositif d'administration locale de médicament non implantable est sélectionné parmi un ballonnet d'élution de médicament (DEB), un cathéter d'administration locale de médicament et tout autre dispositif médical capable d'administrer localement MYH11-pARNi, CNN1-pARNi, LMOD-pARNi, SMTN-pARNi, TPM-pARNi, CALD-pARNi, ACTN-pARNi, ACTA-pARNi et ACTB-pARNi.

15. Dispositif médical d'élution destiné à être utilisé dans le procédé selon la revendication 11, dans lequel ledit vecteur d'inactivateur de gène est choisi parmi un groupe constitué de lentivirus et de plasmides, de nanoparticules, de fluoropolymères, de polymères biorésorbables, de polymères non résorbables, ou peut même être sans polymère (sans aucun type de polymère).

16. Dispositif médical d'élution destiné à être utilisé dans le procédé selon la revendication 10, dans lequel ledit inactivateur de gène est administré seul ou en combinaison avec un autre inactivateur de gène ou avec des médicaments antiprolifératifs cellulaires.

17. Dispositif médical d'élution destiné à être utilisé dans le procédé selon la revendication 10, dans lequel la concentration d'inactivateur de gène est comprise entre 1 µg/ml et 1000 µg/ml, ce qui est une large plage de concentration, puisque l'inactivateur de gène peut être étalé sur la surface du dispositif médical d'une seule couche très mince jusqu'à plusieurs couches épaisses pour atteindre la quantité souhaitée d'inactivateur de gène à administrer localement.

18. Dispositif médical d'élution destiné à être utilisé dans le procédé selon la revendication 10, dans lequel le procédé favorise à la fois la perméabilité permanente du vaisseau sanguin et la ré-endothélialisation précoce d'un segment de vaisseau sanguin traité.
